# EUROPÄISCHE PATENTSCHRIFT

(11) **EP 2 609 210 B1**
(45) Veröffentlichungstag und Bekanntmachung des Hinweises auf die Patenterteilung: **24.06.2015**
(21) Anmeldenummer: 10763588.0
(22) Anmeldetag: 27.08.2010
(51) Int. Cl.: C12Q 1/34

(54) **VERFAHREN ZUR BESTIMMUNG DER ENZYMAKTIVITÄT DER HEPARANASE**
METHOD FOR HEPARANASE ACTIVITY DETERMINATION
MÉTHODE POUR LA DÉTERMINATION D'ACTIVITÉ DE HEPARANASE

(43) Veröffentlichungstag der Anmeldung: 03.07.2013
(73) Patentinhaber: Christian-Albrechts-Universität zu Kiel, 24118 Kiel (DE)
(72) Erfinder: ALBAN, Susanne, 24146 Kiel (DE); SCHIEMANN, Simone, 24118 Kiel (DE); LÜHN, Susanne, 24118 Kiel (DE)
(74) Vertreter: Steinecke, Peter
(86) Internationale Anmeldenummer: PCT/DE2010/000992
(87) Internationale Veröffentlichungsnummer: WO 2012/025069

(56) Entgegenhaltungen:
- WO-A2-00/77241
- HAMMOND E ET AL: "Development of a colorimetric assay for heparanase activity suitable for kinetic analysis and inhibitor screening" ANALYTICAL BIOCHEMISTRY, ACADEMIC PRESS INC, NEW YORK LNKD- DOI:10.1016/J.AB.2009.09.007, Bd. 396, Nr. 1, 1. Januar 2010 (2010-01-01), Seiten 112-116, XP026771205 ISSN: 0003-2697 [gefunden am 2009-09-11] in der Anmeldung erwähnt
- LUEHN SUSANNE ET AL: "Development and evaluation of a fluorescence microplate assay for quantification of heparins and other sulfated carbohydrates" Mai 2010 (2010-05), JOURNAL OF PHARMACEUTICAL AND BIOMEDICAL ANALYSIS, VOL. 52, NR. 1, PAGE(S) 1-8 , XP002609366 ISSN: 0731-7085 in der Anmeldung erwähnt Seite 2, rechte Spalte - Seite 3, rechte Spalte
- FREEMAN C ET AL: "A RAPID QUANTITATIVE ASSAY FOR THE DETECTION OF MAMMALIAN HEPARANASE ACTIVITY" BIOCHEMICAL JOURNAL, THE BIOCHEMICAL SOCIETY, LONDON, GB, Bd. 325, Nr. 1, 1. Juli 1997 (1997-07-01), Seiten 229-237, XP001079249 ISSN: 0264-6021 in der Anmeldung erwähnt
- Schiemann S and Alban S: "Detecting heparanase inhibitors with a novel heparanase assay fit for routine" 2009, XP002609367 Gefunden im Internet: URL:http://www.gth2009.org/fileadmin/templ ate/abstracts/PP5.4-6.PDF [gefunden am 2010-11-12]

## Beschreibung

Die Erfindung betrifft ein Verfahren zur Bestimmung der Aktivität der Heparanase

Das Enzym Heparanase, auch Heparansulfat-Endoglycosidase oder Endo-β-D-glucuronidase genannt, ist eine Endoglycosidase, die spezifisch Heparansulfat-Ketten von Zelloberflächen-und Basalmembran-Heparansulfat-Proteoglycanen spaltet und an zahlreichen wichtigen biologischen Prozessen, wie z.B. Entzündungsreaktionen, aber auch bei der Tumor-Invasion und Metastasierung, beteiligt ist.

Aufgrund seiner zentralen Rolle bei der Invasion und Metastasierung von Tumoren werden daher Inhibitoren der Heparanase als potenzielle Kandidaten für die Tumortherapie angesehen.

Als schwierig erweist sich jedoch das Screening nach wirkungsvollen Heparanaseinhibitoren, da keine für ein Screening mit hohem Durchsatz geeigneten Testverfahren zur Bestimmung der Enzymaktivität der Heparanase zur Verfügung stehen.

In herkömmlichen Assays wird die Heparanaseaktivität bzw. -hemmung anhand des Abaus des ntürlichen Substrates Heparansulfat bestimmt (Nakajima et al 1986). Das hierbei verwendete Heparansulfat ist ein komplex und sehr variabel zusammengesetztes Glycaosaminoglycan und daher in seiner Zusammensetzung schwierig zu standardisieren. Außerdem muss es zum Nachweis der Enzymaktivität beispielsweise mit einem radioaktiven Marker markiert werden (Freeman et al 1997).

Auch neuere Verfahren, die nicht radioaktiv markiertes Substrat, sondern Hapransulfat verwenden, das beispielsweise mit Biotin oder fluoreszierenden Markern (z.B. FITC oder Europium-Chelaten) gekoppelt ist, sind arbeits- und zeitaufwändig, da die Spaltprodukte jeweils abgetrennt werden müssen (Behzad et al 2003; Huang et al 2004).

Darüber hinaus weisen alle Markierungsverfahren den Nachteil auf, dass nicht nur das Heparansulfat selbst, sondern auch die Markierung chargenabhängigen Schwankungen unterliegt und dass die biochemischen Eigenschaften des Substrates und damit sein Verhalten im Messsystem durch die Markierung verändert werden können.

Es ist bekannt, dass neben dem natürlich vorkommenden Heparansulfat auch das synthetisch hergestellte Fondaparinux von der Heparanase gespalten wird und daher als Alternative zu markiertem Heparansulfat als Substrat der Heparanase verwendet werden kann.

Fondaparinux ist ein Arzneistoff, der wie Heparine als Antithrombotikum eingesetzt wird. Es handelt sich um ein chemisch definiertes, synthetisch hergestellters sulfatiertes Pentasaccharid, das der natürlichen Pentasaccharidsequenz entspricht, die auch in Heparinmolekülen vorkommt. Fondaparinux ist ein sogenannter indirekter selektiver Faktor Xa-Inhibitor; es wirkt antithrombotisch, indem es mit hoher Affinität selektiv an Antithrombin (AT) bindet und dessen Hemmwirkung gegenüber dem Gerinnungsfaktor Xa ca. 340fach beschleunigt); diese AT-vermittelte Inhibierung von Faktor Xa bewirkt eine verminderte Bildung von Thrombin, dem Schlüsselenzym der Gerinnung.

Die Heparanase spaltet das Fondaparinux in ein Di- und ein Trisaccharid:

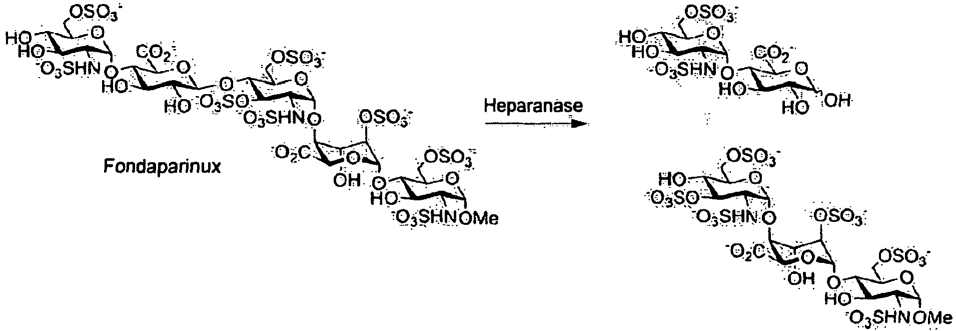

Hierzu ist beispielsweise ein Verfahren bekannt, bei dem die reduzierenden Eigenschaften des Disaccharids mit Hilfe des Tatrazoliumsalzes WST-1, das zu einem bei 584 nm vermessbarem Farbstoff oxidiert wird, genutzt werden (Hammond et al 2010).

Allerdings ist die Nutzbarkeit dieses Verfahren in der Praxis sehr begrenzt, da dieses System, insbesondere die Farbreaktion, extrem störanfällig ist.

Ein auch für das Screening von potenziellen Heparanase-Inhibitoren durchführbares Verfahren besteht derzeit darin, den Abbau von Fondaparinux indirekt, und zwar mittels dessen inhibitorischer Aktivität auf den Gerinnungsfaktor Xa zu ermitteln, wobei die nach Inkubation von Fondaparinux mit Heparanase messbare Abnahme der inhibitorischen Aktivität des Fondaparinux auf Faktor Xa ein Maß für die Enzymaktivität der Heparanase ist (Alban et al 2009).

Allerdings ist dieses Verfahren mit der indirekten Qantifizierung von Fondaparinux, trotz guter Ergebnisse, nicht ideal als robuster Test für ein Hochdurchsatz-Screening von potenteillen Inhibitoren oder Routinemessungen. Denn die indirekte Quantifizierung des Substrates anhand einer biologischen Aktivität umfasst mehrere Schritte, benötigt eine Kalibrierung des Systems und unterliegt gewissen Störfaktoren.

Aufgabe der vorliegenden Erfindung ist es daher, ein einfacheres und schneller durchzuführendes Verfahren zur Bestimmung der Heparanaseaktivität zu schaffen.

Diese Aufgabe wird durch das Verfahren mit den Merkmalen von Anspruch 1 gelöst. Die Unteransprüche geben vorteilhafte Ausgestaltungen der Erfindung wieder.

Grundgedanke der Erfindung ist es, einen Assay zur Bestimmung der Enzymaktivität des Enzyms Heparanase bereitzustellen, bei dem Fondaparinux als Substrat verwendet und direkt mit der fluoreszierenden Sensor-Substanz Polymer-H quantifiziert wird.

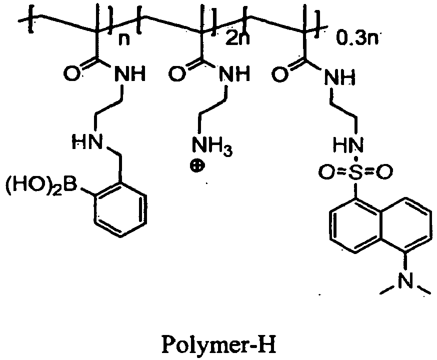

Es ist zwar bekannt, dass das synthetisch hergestellte Fondaparinux, obgleich es im Verhältnis zu anderen Glycosaminoglycanen ein sehr kurzkettiges Pentasaccharid ist, die Fluoreszenzintensität von Polymer-H konzentrationsabhängig steigert und somit mittels dieser Sensor-Substanz detektiert werden kann (Lühn et al 2010).

Überraschend ist jedoch, dass die nach Inkubation von Fondaparinux mit Heparanase vorliegenden Fondaparinux-Spaltprodukte keine Steigerung der Fluoresenzintensität von Polymer-H mehr hervorrufen, wobei in Voruntersuchungen zur vorliegenden Erfindung gezeigt werden konnte, dass nach Inkubation Fondaparinux mit Heparinase die Fluoresenzintensität bis zu einer bestimmten Menge Substrat ungefähr auf den Kontrollwert ohne Fondaparinux absinkt und erst ab einer bestimmten Substratmenge ansteigt.

Die Erfindung wird anhand der in den beigefügten Abbildungen dargestellten experimentellen Ergebnisse näher erläutert. Es zeigen:
- Fig. 1: die Konzentrationsabhängigkeit der Steigerung der Polymer-H-Fluoreszenzintensität durch Fondaparinux;
- Fig. 2: die Abhängigkeit des Abbaus von Fondaparinux (FPX) von der Heparanase-Konzentration und der Inkubationszeit;
- Fig. 3: die Abhängigkeit des Abbaus von Fondaparinux (FPX) von der Heparanase-Konzentration nach jeweils 30 min;
- Fig. 4: die Abhängigkeit des Abbaus von Fondaparinux (FPX) von der Fondaparinux- und Heparanasekonzentration
- Fig. 5: den Einfluss von Calcium-Ionen auf die Heparanaseaktivität;
- Fig. 6: den Einfluss von Antithrombin (AT) auf den Abbau von Fondaparinux durch Heparanase;
- Fig. 7: den Einfluss verschiedener Assaykomponenten auf die gemessene Fluoreszenzintensität; und

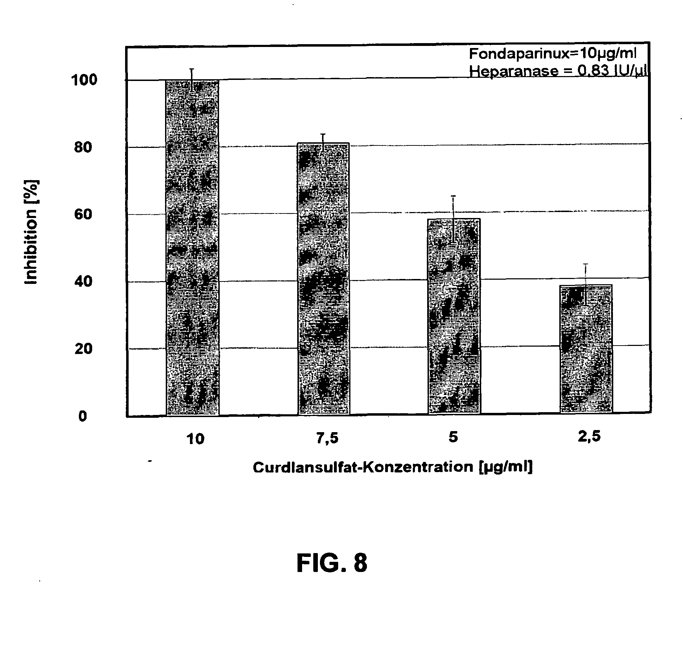

- Fig. 8: die konzentrationsabhängige Hemmung der Heparanase durch Curdlansulfat.

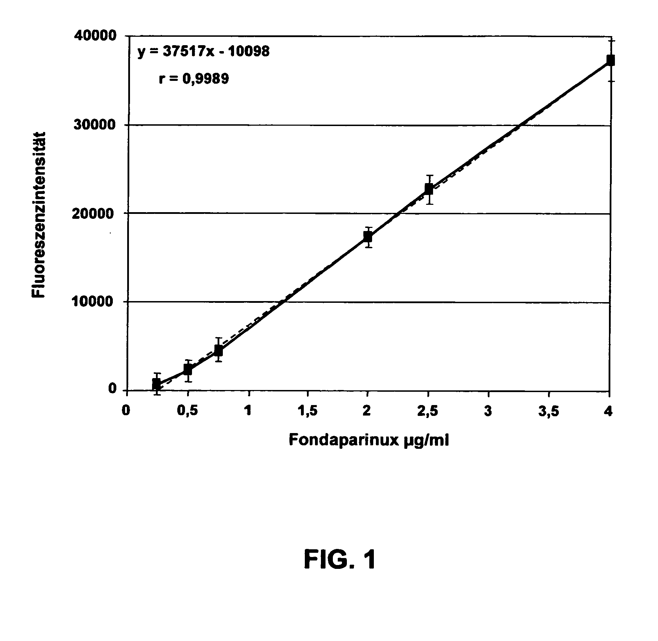

Fig. 1 zeigt den Zusammenhang zwischen der Konzentration von Fondaparinux und der mit Polymer-H erhaltenen Fluoreszenzintensität. Es ist deutlich zu erkennen, dass die Zunahme der Fluoreszenzintensität bei zunehmenden Konzentrationen linear verläuft und damit für eine zuverlässige Quantifizierung in einem Verfahren zur Bestimmung der Heparanaseaktivität geeignet ist.

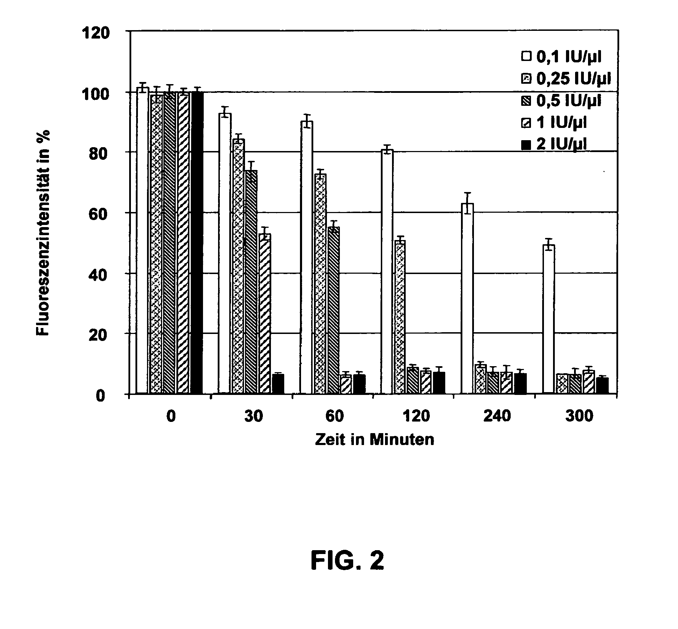

Fig. 2 gezeigte Versuchsergebnis wurden zunächst wurden 15 µl einer 100 µg/ml Fondaparinux-Lösung in die Wells einer MTP pipettiert, anschließend 135 µl Heparanase-Lösung unterschiedlicher Aktivität zugegeben. Nach Inkubation bei 37 °C für die jeweils angegebene Zeit wurden 100 µl Aliquots abpipettiert und zur Inaktivierung der Heparanase 3min lang bei 100 °C inkubiert. Anschließend erfolgte die Fluoreszenzmessung nach Zugabe von Polymer-H-Lösung.

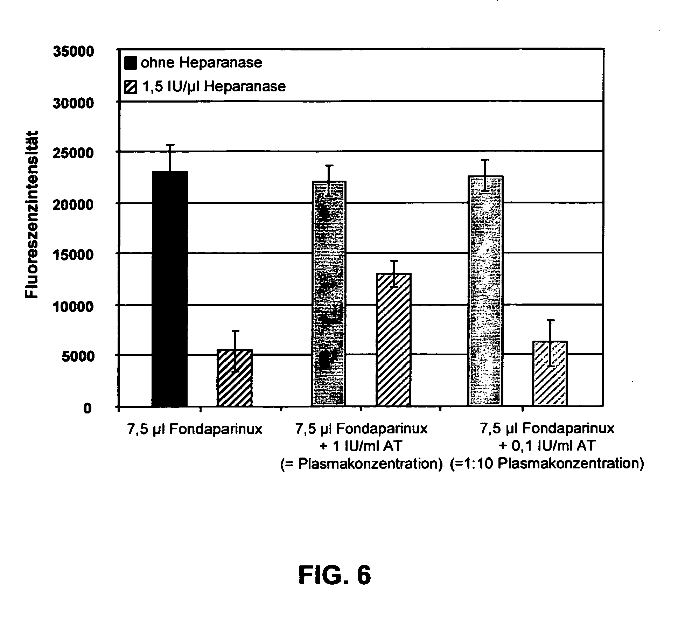

Fig. 2 zeigt, dass der Abbau einerseits von der Inkubationszeit, andererseits von der Heparinase-Konzentration abhängt. Während die höchste Heparanase-Konzentration (2,0 IU/µl) innerhalb von 30 min für einen vollständigen Abbau von Fondaparinux sorgt, ist er mit 0,25 IU/µl nach 240 min erreicht. Die "Restfluoreszenz" entspricht der der Kontrolllösung ohne Fondaparinux (vgl. Fig. 6).

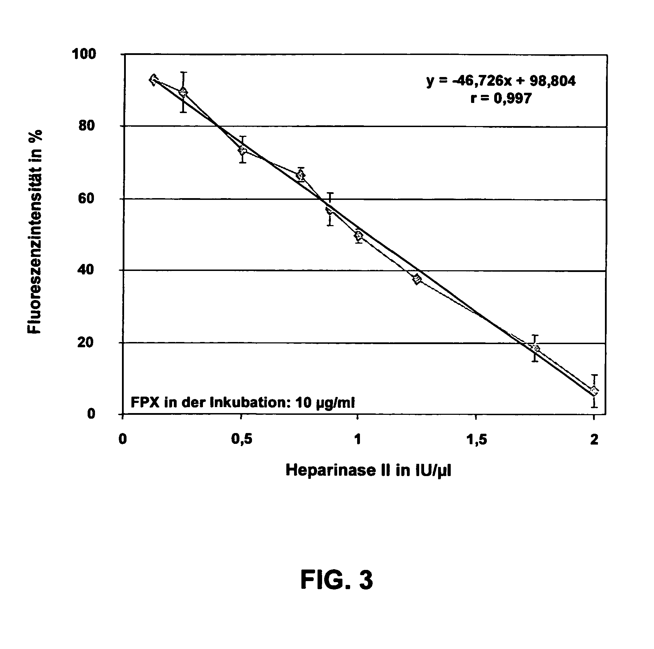

Aufgrund des in Fig. 2 dargestellten Ergebnisses sollte geprüft werden, in welchem Heparanase-Konzentrationsbereich der Abbau von Fondaparinux und damit die Fluoreszenzintensität eine lineare Abhängigkeit von der Enzymkonzentration zeigt. Wie in Fig. 3 zu sehen, ergaben Heparanase-Konzentrationen von 0,1 bis 2,0 IU/µl einen unvollständigen Fondaparinux-Abbau, wobei deutlich jedoch zu erkennen ist, dass die Fluoreszenzintensität linear mit der Heparanase-Konzentration abnimmt.

Aufgrund dieses linearen Zusammenhangs ergibt sich die Option, mit einer Referenz-Heparanase eine Kalibriergerade zu erstellen und anhand dieser die Heparanase-Aktivität einer unbekannten Probe zu bestimmen.

Da bei geringen Heparanase-Konzentrationen nach einer bestimmten Inkubationszeit ein erheblicher Anteil von Fondaparinux erhalten bleibt und der Abbau nicht messbar ist, sollte untersucht werden, ob eine Verringerung der Substratkonzentration die Empfindlichkeit steigert. Hierzu wurden die Heparanase-Konzentrationen 0,1, 0,5 und 1,0 IU/µl jeweils 30 min mit 10,0 µg/ml Fondaparinux und den geringeren Konzentrationen 5,0 und 2,5 mg/ml inkubiert. Für die Fluoreszenzmessung nach Polymer-H-Zugabe wurden die Inkubationslösungen dann 1:4, 1:2 verdünnt bzw. unverdünnt verwendet (d.h. einheitlich 2,5 µg/ml Fondaparinux).

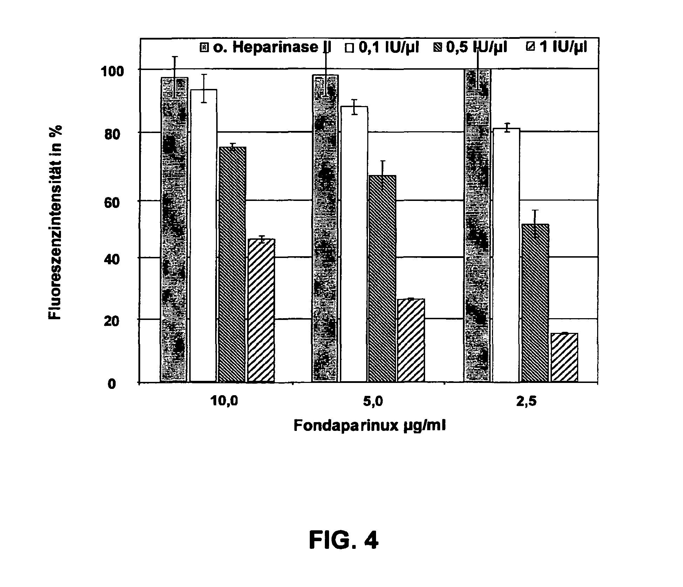

Fig. 4 zeigt, dass durch Verringerung der Substratkonzentration der Messbereich zu niedrigeren Heparinase-Konzentrationen verschoben werden kann. Angesichts des linearen Konzentrationsbereiches der Fluoresenzintensitätssteigerung von Polymer-H durch Fondaparinux können auch noch geringere Konzentrationen zwecks Steigerung der Empfindlichkeit eingesetzt werden.

Bekanntlich wird die Heparanase-Aktivität gegenüber Heparansulfat durch Calcium-Ionen gesteigert, Daher wurde untersucht, ob dies auch bei Verwendung von Fondaparinux als Substrat möglich ist. Dazu wurde die als Standard festgelegte Heparanase-Arbeitslösung (1,5 IU/µl) zum einen mit der 10 mM CaCl₂-Lösung, zum anderen mit dem Standardpuffer 1:2 verdünnt (0,75 IU/µl).

Wie Fig. 4 zeigt, steigert CaCl₂ die Heparanaseaktivität um fast 100 %. Dies demonstriert beispielhaft, dass nicht nur durch eine Verlängerung der Inkubationszeit, sondern auch durch Modifiaktion des Inkubationsbuffers auch sehr geringe Heparanase-Konzentrationen nachgewiesen werden.

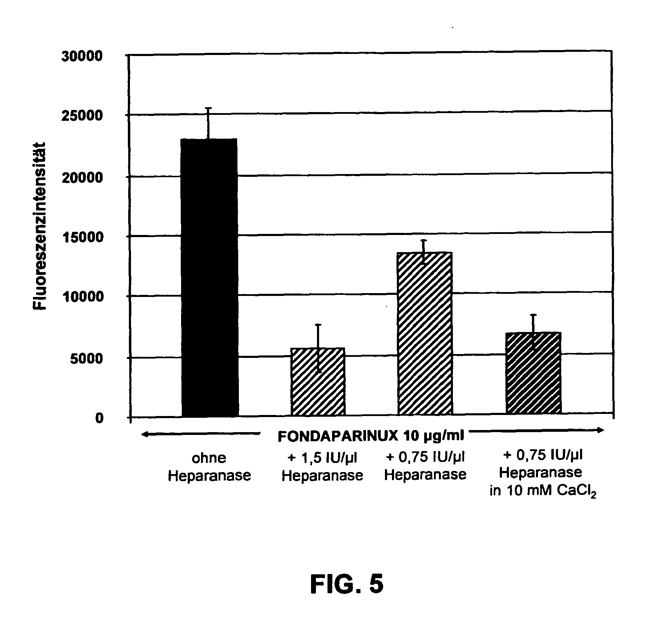

Für den in Fig. 5 dargestellten Versuch wurden zu gleichen Volumenanteilen 1 IU/ml Antithrombin bzw. 0,1 IU/ml Antithrombin und 200 µg/ml Fondaparinux (je 7,5 µl) mit 135 µl Heparanase-Lösung (1,5 IU/µl) inkubiert. Die Antithrombin-Konzentration von 1,0 IU/ml, die der Antithrombin-Konzentration im Plasma entspricht, stört den Abbau von Fondaparinux. Wird das Antithrombin aber um 1:10 auf 0,1 IU/ml verdünnt (entspricht einer Plasmaverdünnung von 1:10), kann der Einfluss des Antithrombin auf den Assay eliminiert werden.

In Fig. 6 sind die gemessenen FI-Werte der verschiedenen Assay-Komponenten dargestellt, d.h. ohne Abzug des Blank-Wertes. Die Angabe der Konzentration bezieht sich auf die Inkubationslösung; die Konzentration von Fondaparinux bei der Fluoreszenzmessung beträgt 2,5µg/ml.

Der bei der Inkubation verwendete Natriumacetat-Puffer ("NaAc") hat keinerlei Einfluss auf die Fluoreszenzintensität (FI) von Polymer-H.

Heparanase führt zu einer geringfügigen Erhöhung der Fluoreszenzintensität, die sich auch in der Fluoreszenzintensität von "FPX ink." (Probe nach Heparanase-Zugabe sofort inaktiviert, so dass Fondaparinux nicht abgebaut wird) im Vergleich zu einer Fondaparinux-Lösung ohne Heparanase manifestiert.

Die nach Abbau von Fondaparinux gemessene Fluoreszenzintensität ist nahezu gleich mit der von "NaCl + Heparanase" und veranschaulicht einen vollständigen Abbau.

Keinerlei Einfluss auf die Fluoreszenzintensität von Polymer-H zeigt Galactose (2,5 µg/ml). Hieraus ergibt sich ein Vorteil gegenüber dem Heparanase-Assay von Hammond (s.o.). Dort führt Galactose in der Detektionsreaktion zu einem Signal, das identisch mit dem der Fondaparinux-Spaltprodukte ist, denn jegliche Art reduzierende und oxidierender Komponenten stört diesen Test.

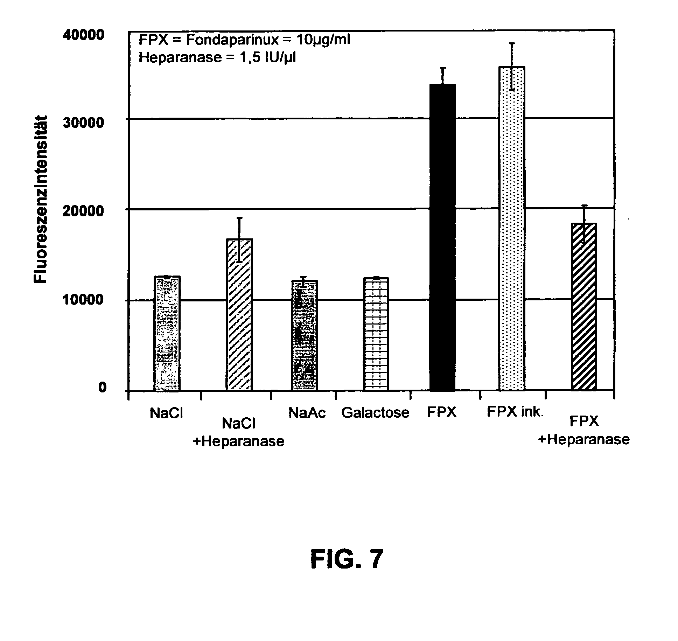

Schließlich zeigt Fig. 7 exemplarisch das Ergebnis eines Versuchs zur Hemmung der Heparanase-Aktivität durch einen bekannten Heparanase-Inhibitor, nämlich Curdlansulfat. Hierzu wurden der Inhibitor der üblichen Inkubationslösung zugesetzt. Testschema: 7,5 µl einer 200 µg/ml FPXLösung (Endkonz. während der Inkubation 10 µg/ml), 7,5 µl Curdlansulfat-Lösung in 0,9 % NaCl (Endkonz. in Fig. 7 angegeben) und 135µl 1,5 IU/µl Heparanase-Lösung wurden jeweils 30 min bei 37 °C inkubiert, anschließend inaktiviert, verdünnt und nach Polymer-H-Zugabe vermessen.

Gegenüber den vorbekannten Verfahren zur Bestimmung der Enzymaktivität von Heparanase besitzt das erfindungsgemäße Verfahren folgende Vorteile:
- Das hier vorgeschlagene Verfahren ist im Kern eine direkte Quantifizierung des Substrates Fondaparinux mittels Fluoreszenzmessung. Das Verfahren ist damit einfach, schnell und ohne strenge Sicherheitsvorkehrungen durchzuführen.
- Durch die Verwendung von Fondaparinux als Substrat ist der Test erheblich preiswerter, besser standardisierbar und leichter reproduzierbar als Verfahren mit chemisch oder radioaktiv markiertem Heparansulfat.
- Als Verfahren, das in Mikrotiterplatten durchgeführt wird, ist der Test voll vollautomatisierbarer und innerhalb von 45 min inkl Inkubationsreaktion durchführbar.
- Da es sich bei der Detektionsreaktion um eine direkte Bestimmung von Fondaparinux handelt und die Fluoreszenintensität linear von der Fondaparinux-Konzentration. abhängt, braucht man bei der Detektionsreaktion keine Kalibrierkurve, sondern lediglich einen Fondaparinux-Kontrollwert, nämlich die Fluoreszenzintensität von nicht abgebautem Fondaparinux (entspricht 100 % Fluoreszenzintensität).
- Das Verfahren eignet sich nicht nur zum Inhibitor-Screening, sondern auch zur Quantifizierung bekannter Heparanase-Inhibitoren in biologischen Proben (z.B. Blutplasma). Darüber hinaus eignet sich das Verfahren zur Prüfung einer unbekannten Heparanase im Vergleich zu einer Referenz (Qualitätskontrolle) sowie zur Bestimmung der Heparanase-Aktivität in biologischen und Proben anderer Art.
- Da Fondaparinux mit keinen anderen Zellen und Proteinen interagiert als Antithrombin (Petitou et al. 2004), ist der Test breit einsetzbar und der Abbau wird nicht durch "Matrixeffekte" gestört. Zur Abtrennung der Lösung für die Detektion von komplexen Proben (Zelllysate, Zellen, Gewebelysate), können aus diesem Grund auch Ultrafiltrations-Mikrotiterplatten (z.B. von DoctorLab.com) verwendet werden, die nach der Inkubation zentrifugiert werden, wodurch restliches Fondaparinux, dessen Abbauprodukte durch eine Membran mit einem cut-off 5 kDa in eine untere Platte überführt werden und alle größeren Moleküle (z.B. Proteine) und Partikel in der oberen Platte bleiben.
- Obwohl Fondaparinux an Antithrombin im Blutplasma bindet, kann der Test ohne weitere Modifikationen verwendet werden, wenn die Plasmaproben 1:10 (z.B. für das Drug-Monitoring) verdünnt werden.
- Wird jedoch unverdünntes Plasma vermessen, konkurriert die Bindung an Antithrombin mit dem Abbau durch die Heparanase. Um dieses Problem zu lösen, muss Antithrombin in dem Plasma abgefangen werden. Eine Strategie besteht nun darin, dem Plasma Idraparinux zuzusetzen, da dieses chemisch modifizierte Analogon von Fondaparinux 800mal stärker als Fondaprinux an Antithrombin bindet (Hjelm et al. 2007), selbst aber nicht von der Gruppe der Heparanasen abgebaut wird (Daud et al. 2001, unpublizierte Daten). Demzufolge wird Fondaprinux kompetitiv aus der Bindung zu Antihrombin durch Idraparinux verdrängt und kann durch die Heparanase abgebaut werden. Eine andere Option ist das Abfangen von Antithrombin in der Probe durch Zugabe von Antithrombin-Antikörpern.

Das Verfahren kann zur Bestimmung der enzymkinetischen Faktoren, wie K_{M} oder vₘₐₓ, in Anwesenheit oder Abwesenheit von (potenziellen) Inhibitoren verwendet werden. Zur Bestimmung der Enzymaktivität reicht es, beispielsweise für Screening-Tests, aber auch aus, bei bestimmten Konzentrationen von Enzym, Substrat und Inhibitorkandidat Punktmessungen vorzunehmen, um zu überprüfen, ob die Fluoreszenzintensität aufgrund einer Hemmung der Heparanase durch den potenziellen Inhibitor beeinflusst wird - für entsprechende Kontrollansätze weiß der Fachmann zu sorgen.

Ist ein Inhibitor der Heparanase dadurch identifiziert, dass eine niedrigere Fluoreszenzintensitätsänderung eintritt als im Kontrollansatz ohne Inhibitor, wird der Fachmann auch die weiteren Eigenschaften eines mit dem vorgeschlagenen Verfahren identifizierten Inhibitors ohne weiteres gemäß dem Stand der Technik ermitteln können.

### Beispiel 1

### Technische Mittel

### Geräte

- Mikrotiterplatten (MTP)-Reader für Fluoreszenz-Messung:
   z.B. Optima^{®} (BMG LABTECH GmbH), Filter: λₑₓ 320nm und λₑₘ 510 nm
   z.B. Omega^{®} (BMG LABTECH GmbH), Filter: λₑₓ 320nm und λₑₘ 510 nm
   z.B. Novostar^{®} (BMG LABTECH GmbH) mit Titrator; λₑₓ 320nm und λ_{cm} 510 nm
- Flachboden-Mikrotiterplatten (MTP):
   MTP für Inkubationsreaktion: Best.Nr. 269620 (nunc GmbH & Co.KG)
   MTP für Detektionsreaktion: Maxisorp^{®}, Best.Nr. 437111 (nunc GmbH & Co.KG)
- Inkubationsschüttler für Heparanase-Inkubation (37 °C, 600 RPM):
   z.B. Hybridization Oven/Shaker (Amersham Pharmacia biotech, Wien, Österreich)

### Reagenzien

- Fondaparinux:
   Bulkware von Glaxo Smith Kline und Fertigspritze Arixtra^{®} (2,5 mg/0,5 ml) Fondaparinux-Substratlösung: Fondaparinux. wird zu 100 µg/ml oder 200 µg/ml in 0,9 % NaCl gelöst
- Heparanase:
   Heparinase II (Flavobacterium heparinum) (Sigma, Best.Nr. H 6512, Charge: 076K3777 155 Sigma-Units (SU)/mg; 1 SU = 600 Internationale Units (IU)) Arbeitslösung: z.B. 1,2 oder 0,9 SU/ml = 2,0 oder 1,5 IU/µl Heparanase
- Heparanase-Puffer:
   für die Herstellung der Stammlösung: pH 7,0, 100 mM Natriumacetat mit 0,01 % BSA für die Herstellung der Arbeitslösung: pH 7,0, 100 mM Natriumacetat
- Polymer-H
   Synthese nach Sun et al. (2007)
   Arbeitslösung: 75 µg/ml in 0,9 % NaCl

### Weitere Reagenzien:

- humanes Antithrombin (Haemochrom Diagnostics GmbH, Best.Nr. 41220, 10 IU)
- 10 mM Calciumchlorid-Löung (CaCl₂) mit Heparanase-Puffer verdünnt auf 5mM in Inkubationslösung
- Heparanase-Inhibitor: Curdlansulfat, ein semisynthetisches β-1,3-Glucansulfat

### SCHRITT 1: Fondaparinux-Abbau mit Heparanase

In die Wells einer MTP werden zunächst jeweils 7,5 µl der Fondaparinux-Lösung (200 µg/ml) und 7,5 µl der jeweiligen Probenlösung (in den Beispielen: Antithrombin, Inhibitor, 0,9% NaCl dann als Kontrolle) pipettiert oder alternativ 15 µl der Fondaparinux-Lösung (100 µg/ml) (wenn die Heparanase-Aktivität in Proben bestimmt werden soll). Es werden jeweils zwei Wells mit der gleichen Lösung belegt.

Dann werden jeweils 135 µl der Heparanase-Arbeitslösung bzw. ggf. 135 µl einer Heparanasehaltigen Probenlösung zupipettiert, so dass die Substrat-Konzentration 10 µg/ml beträgt. Es folgt eine Inkubation von 30 min bei 37 °C unter Schütteln (600 RPM) (für Kinetik-Untersuchungen wurde die Probe für den 0 min-Wert 1 min bei 37 °C geschüttelt und dann gleich inaktiviert).

Nach Ablauf der Inkubationszeit wird die Heparanase inaktiviert, indem sie z.B. für 3 Minuten bei 100 °C Wasserbad erhitzt wird. Alternativ wird die MTP entweder sofort und zeitlich standardisiert dem Schritt 2 unterzogen (Beispiel, siehe Fig. 5) (automatisiert möglich im Novostar^{®}).

### SCHRITT 2: Fluoreszenzmessung zur Fondaparinux-Detektion

Aus jedem Well werden jeweils zwei 45 µl Aliquots der inkubierten Lösung in zwei Wells einer Maxisorp^{®}-MTP überführt (d.h. insgesamt 4 Werte pro Probe) und jeweils 1:4 mit 135 µl 0,9 % NaCl verdünnt.

Anschließend werden jeweils 20 µl Polymer-H-Arbeitslösung (75 µg/ml) zupipettiert. Dann wird die MTP zunächst 5 min geschüttelt, weitere 5 min bei RT im MTP-Reader äquilibriert und nach nochmaligem Schütteln für 30 s von oben vermessen (λₑₓ 320-10nm, λₑₘ 510-10 nm).

### Beispiel 2

Beispiel 2 unterscheidet sich von Beispiel 1 dadurch, dass beide Schritte in einer Mikrotiterplatte durchgeführt werden. Dieses Vorgehen ist besonders für die Automatisierung geeignet (z.B. bei Verwendung des Novostar^{®})

### Arbeitsschritte:

- 3,0 µl bzw. 6,0 µl der Fondaparinux-Lösung (200 bzw. 100 µg/ml)
- 3,0 µl bzw. 0,0 µl Probenlösung
- 54 µl der Heparanase-Arbeitslösung
- Inkubation
- 180 µl 0,9 % NaCl
- 25 µl Polymer-H-Arbeitslösung (80 µg(ml)
- Fluoreszenzmessung

### Beispiel 3

Beispiel 3 unterscheidet sich von Beispiel 2 dadurch, dass eine geringere Substratkonzentration verwendet wird, die vor der Polymer-H-Zugabe nicht verdünnt wird. Außerdem wird in diesem Beispiel nicht die Hemmung des Abbaus untersucht, sondern die Aktivität einer Heparanase-haltigen Probe anhand einer Kalibriergerade mit verschiedenen Konzentrationen einer Referenz-Heparanase.

### Arbeitsschritte:

- 20,0 µl der Fondaparinux-Lösung (25 µg/ml)
- 180 µl der Referenz-Heparanase-Lösung und Probelösung
- Inkubation
- 22 µl Polymer-H-Arbeitslösung (75 µg(ml)
- Fluoreszenzmessung

### Literatur

Alban S, Schiemann S. Development of a novel heparanase activity assay and identification of potent heparanase inhibitors. XII Congress of the International Society on Thrombosis and Haemostasis, Boston, 11.-16.07.2009. Abstract publiziert in: J Thromb Haemost (2009) 7, Suppl.2: PP-WE-506.
Bisio A, Mantegazza A, Urso E, Naggi A, Torri G, Viskov C, Casu B. High-performance liquid chromatographic/mass spectrometric studies on the susceptibility of heparin species to cleavage by heparanase. Semin Thromb Hemost. 2007 Jul;33(5):488-95.
Freeman C, Parish CR. A rapid quantitative assay for the detection of mammalian heparanase activity. Biochem J. 1997 Jul 1;325 (Pt 1):229-37.
Hammond E, Li CP, Ferro V. Development of a colorimetric assay for heparanase activity suitable for kinetic analysis and inhibitor screening. Anal Biochem. 2010 Jan 1;396(1):112-6.
Huang KS, Holmgren J, Reik L, Lucas-McGady D, Roberts J, Liu CM, Levin W. Highthroughput methods for measuring heparanase activity and screening potential antimetastatic and anti-inflammatory agents. Anal Biochem. 2004 Oct 15;333(2):389-98.
Lühn S, Schrader T, Sun W, Alban S.Development and evaluation of a fluorescence microplate assay for quantification of heparins and other sulfated carbohydrates. J Pharm Biomed Anal. 2010 May 1;52(1):1-8. Epub 2009 Dec 23.
S Lühn, T Schrader, W Sun, S Alban. Development and evaluation of a fluorescence microplate assay for quantification of heparins and other sulfated carbohydrates. J Pharm Biomed Anal 52 (2010): 1-8
Nakajima M, Irimura T, Nicolson GL. A solid-phase substrate of heparanase: its application to assay of human melanoma for heparan sulphate degradative activity. Anal Biochem. 1986 Aug 15;157(1):162-71.
Schiemann S. Entwicklung eines Heparinase II-Testes. Diplomarbeit, CAU Kiel, 2008.
Schiemann S, Alban S. Detecting heparanase inhibitors with a novel heparanase activity assay fit for routine. 53rd Annual Meeting of the GTH (Gesellschaft für Thrombose- und Hämostaseforschung), Wien, 04.-07.02.2009. Abstract publiziert in: Hämostaseologie (2009) 29: A73 (PP6.6-3).

## Patentansprüche

1. Verfahren zur Bestimmung der Enzymaktivität der Heparanase,
**gekennzeichnet durch** die Schritte
- Inkubieren einer Fondaparinux und Polymer-H enthaltenden Lösung mit Heparanase, und
- Erfassen der Veränderung der Fluoreszenzintensität des Polymer-H innerhalb wenigstens eines vorbestimmten Zeitintervalls, wobei die Fluoreszenzintensitätsänderung ein direktes Maß für die Enzymaktivität der Heparanase ist.

2. Verfahren nach Anspruch 1, **dadurch gekennzeichnet, dass** die Lösung eine die Aktivität der Heparanase steigernde Substanz enthält.

3. Verfahren nach Anspruch 2, **dadurch gekennzeichnet, dass** die Lösung Ca²⁺-Ionen enthält.

4. Verfahren nach einem der vorhergehenden Ansprüche, **dadurch gekennzeichnet, dass** die Lösung eine 1:10 verdünnte Plasmalösung ist.

5. Verfahren nach einem der vorhergehenden Ansprüche, **dadurch gekennzeichnet, dass** die Lösung Idraparinux enthält.

6. Verfahren nach einem der vorhergehenden Ansprüche, **dadurch gekennzeichnet, dass** die Lösung einen Antithrombin-Antikörper enthält.

7. Verfahren nach einem der vorhergehenden Ansprüche, **dadurch gekennzeichnet, dass** die Lösung eine die Enzymaktivität der Heparanase inhibierende Substanz enthält.

8. Verfahren nach einem der vorhergehenden Ansprüche, **dadurch gekennzeichnet, dass** die Heparanase in gelöster Form vorliegt.

9. Verfahren nach einem der vorhergehenden Ansprüche, **dadurch gekennzeichnet, dass** die Heparanase aus einer biologischen Probe stammt.

10. Verfahren nach einem der vorhergehenden Ansprüche, **dadurch gekennzeichnet, dass** die Fluoreszenzintensität bei einer Emissionswellenlänge von 510 nm erfasst wird.

11. Verfahren nach einem der vorhergehenden Ansprüche, **dadurch gekennzeichnet, dass** die Veränderung der Fluoreszenzintensität bei Licht mit einer Anregungswellenlänge von 320 nm erfasst wird.

## Claims

1. A method for determining the enzyme activity of the heparanase,
**characterized by** the steps
- incubating a solution containing fondaparinux and polymer-H with heparanase, and
- detecting the change in fluorescence intensity of the polymer-H within at least one predetermined time interval, wherein the fluorescence intensity change is a direct measure of the enzyme activity of the heparanase.

2. The method according to claim 1, **characterized in that** the solution contains a substance enhancing the activity of the heparanase.

3. The method according to claim 2, **characterized in that** the solution contains Ca²⁺ ions.

4. The method according to any one of the preceding claims, **characterized in that** the solution is a 1:10 diluted plasma solution.

5. The method according to any one of the preceding claims, **characterized in that** the solution contains idraparinux.

6. The method according to any one of the preceding claims, **characterized in that** the solution contains an anti-thrombin antibody.

7. The method according to any one of the preceding claims, **characterized in that** the solution contains a substance inhibiting the enzyme activity of the heparanase.

8. The method according to any one of the preceding claims, **characterized in that** the heparanase is present in dissolved form.

9. The method according to any one of the preceding claims, **characterized in that** the heparanase is derived from a biological sample.

10. The method according to any one of the preceding claims, **characterized in that** the fluorescence intensity is detected at an emission wavelength of 510 nm.

11. The method according to any one of the preceding claims, **characterized in that** the change in fluorescence intensity is detected at light with an excitation wavelength of 320 nm.

## Revendications

1. Une méthode de détermination de l'activité enzymatique de l'héparanase, **caractérisée par** les étapes suivantes:
- Incubation d'une solution contenant du fondaparinux et le polymère-H avec de l'héparanase et
- Détection de la modification d'intensité du polymère H à l'intérieur d'au moins un intervalle de temps prédéterminé, dans laquelle la modification d'intensité est une mesure directe de l'activité enzymatique de l'héparanase.

2. La méthode selon la revendication 1, **caractérisée par le fait que** la solution contient une substance renforçant l'activité de l'héparanase.

3. La méthode selon la revendication 2, **caractérisée par le fait que** la solution contient des ions Ca²⁺.

4. La méthode selon l'une quelconque des revendications précédentes, **caractérisée par le fait que** la solution est une solution de plasma diluée à 1 :10.

5. La méthode selon l'une quelconque des revendications précédentes, **caractérisée par le fait que** la solution contient de l'idraparinux.

6. La méthode selon l'une quelconque des revendications précédentes, **caractérisée par le fait que** la solution contient un anticorps antithrombine.

7. La méthode selon l'une quelconque des revendications précédentes, **caractérisée par le fait que** la solution contient une substance inhibant l'activité enzymatique de l'héparanase.

8. La méthode selon l'une quelconque des revendications précédentes, **caractérisée par** la présence de l'héparanase en solution.

9. La méthode selon l'une quelconque des revendications précédentes, **caractérisée par le fait que** l'héparanase est dérivée d'un échantillon biologique.

10. La méthode selon l'une quelconque des revendications précédentes, **caractérisée par le fait que** l'intensité de la fluorescence est détectée à une longueur d'onde d'émission de 510 mm.

11. La méthode selon l'une quelconque des revendications précédentes, **caractérisée par le fait que** le changement d"intensité de la fluorescence est détecté à la lumière par une longueur d'onde d'excitation de 320 mm.
